(19) Europäisches Patentamt
European Patent Office
Office européen des brevets

(11) EP 1 086 992 B1

(12) EUROPÄISCHE PATENTSCHRIFT

(45) Veröffentlichungstag und Bekanntmachung des Hinweises auf die Patenterteilung:
**14.04.2004 Patentblatt 2004/16**

(51) Int Cl.[7]: **C09B 29/036**, C09B 29/36, C09B 27/00, C09B 45/14

(21) Anmeldenummer: **00119348.1**

(22) Anmeldetag: **08.09.2000**

# (54) Mono-und Dikaliumsalze von Azoverbindungen

Mono- and dipotassium salts of azocompounds

Mono- et Disels de potassium de composés azoiques

(84) Benannte Vertragsstaaten:
**CH DE ES FR GB IT LI**

(30) Priorität: **21.09.1999 DE 19945245**

(43) Veröffentlichungstag der Anmeldung:
**28.03.2001 Patentblatt 2001/13**

(73) Patentinhaber: **Bayer Chemicals AG**
**51368 Leverkusen (DE)**

(72) Erfinder:
- **Sommer, Richard, Dr.**
  **51519 Odenthal (DE)**
- **Linke, Frank**
  **51069 Köln (DE)**
- **Herrmann, Udo, Dr.**
  **41541 Dormagen (DE)**

(56) Entgegenhaltungen:
**EP-A- 0 074 515**
**EP-A- 0 093 306**
**EP-A- 0 297 397**
**DE-A- 2 064 093**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft die Mono- und Dikaliumsalze von Azoverbindungen der Formel (I) sowie deren Hydrate, Verfahren zu ihrer Herstellung, ihre Verwendung zur Herstellung von Metallkomplexen, Substrate, enthaltend die auf diese Weise erhältlichen Metallkomplexe sowie Kaliummetallkomplexe, die wenigstens eine Verbindung eingelagert enthalten.

Die Verwendung von Azobarbitursäure sowie deren Metallsalze, vor allem solche mehrwertiger Metalle, als Pigmente ist bereits in DE-A-2 064 093 beschrieben. Als geeignetes Herstellungsverfahren für die Salze mehrwertiger Metalle wird die Umsetzung von Barbitursäure mit einem Azogruppenüberträger, z.B. Benzolsulfonylazid im alkalischen Medium nach der von Regnitz (Angewandte Chemie, 79, 786 (1967)) beschriebenen Methode, in Gegenwart entsprechender Metallsalze oder die Umsetzung von Azobarbitursäure mit Salzen mehrwertiger Metalle beschrieben.

Gemäß EP-A-297 397 werden zur Herstellung von Diazoverbindungen von α,γ-Diketonen, wie z.B. Barbitursäure, als Azogruppenüberträger Azidoformamidiniumsalze eingesetzt. Unter anderem wird hier auch die Herstellung der entsprechenden Azoverbindungen wie beispielsweise Azobarbitursäure durch Kupplung der so hergestellten Diazobarbitursäure mit Barbitursäure in natriumalkalischem Medium beschrieben.

Gemäß EP 93 306 und EP-A-994164 werden bereits Azobarbitursäurederivate zur Herstellung von entsprechenden Azo-Pigmenten eingesetzt.

Gemäß EP-A-73 463 werden Interkalationsverbindungen der Metallsalze von Azoderivaten bestimmter α, γ-Diketone unter anderem auch von Barbitursäure und deren Verwendung als Pigmente offenbart. Als Herstellungsverfahren der Azobarbitursäure wird die Umsetzung von Barbitursäure mit einem Azogruppenüberträger in neutralem bzw. alkalischem Medium nach der von Regnitz beschriebenen Methode (s. o.) mit oder ohne Zwischenisolierung der zunächst entstehenden Diazobarbitursäure offenbart. Als Azogruppenüberträger wird jedoch ausschließlich Natriumnitrit eingesetzt, wodurch die Azobarbitursäure als Natriumsalz anfällt. Das nach den in der Literatur beschriebenen Synthesewegen hergestellte Natriumsalz der Azobarbitursäure enthält erhebliche Mengen von bis zu 15 Mol-% an nicht umgesetzter Diazobarbitursäure eingeschlossen. Als Verunreinigung des Azobarbitursäurenatriumsalzes führt die Diazobarbitursäure bei der anschließenden Umsetzung mit anderen Metallsalzen, insbesondere mit Nickelsalzen (Herstellung von C.I. Pigment Yellow 150), und Interkalierung ebenfalls zu unerwünschten Nebenprodukten, was zu verunreinigten Pigmenten führt.

Überraschenderweise wurde nun gefunden, dass die Mono- sowie Dikaliumsalze, die der Formel (IIa) oder einer ihrer tautomeren Strukturen entsprechen,

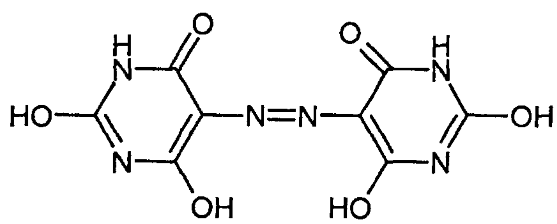

(IIa),

sowie ihre Hydrate, diese Nachteile nicht aufweisen. Die erfindungsgemäßen Salze enthalten vorzugsweise keine bzw. nur vernachlässigbare Mengen an Diazobarbitursäure.

Ebenfalls überraschend war es, dass die Mono- wie Dikaliumsalze der Formel (IIa), insbesondere aber das Monokaliumsalz der Azobarbitursäure der Formel (IIa) auch wesentlich besser geeignet ist für die Synthese der entsprechenden Metallkomplexe der Azobarbitursäure und deren Interkalationsverbindungen, beispielsweise C.I. Pigment Yellow 150 als das üblicherweise verwendete Natriumsalz, da das Verfahren sehr viel schneller abläuft.

Ein weiterer Vorteil der erfindungsgemäßen Mono- bzw. Dikaliumsalze besteht darin, dass unter den üblichen Synthesebedingungen stets reproduzierbare Produktqualitäten erhalten werden, während dies bei den entsprechenden Natriumsalzen nicht der Fall ist. Da die Produktqualität auf der Eduktseite sich auf die Produktqualität der daraus resultierenden Pigmente durch Umsetzung mit entsprechenden Metallsalzen auswirkt, haben die erfindungsgemäßen Kaliumsalze auch unmittelbare Einflüsse auf die daraus resultierenden Pigmente. Somit sind die erfindungsgemäßen Mono- oder Dikaliumsalze gegenüber den üblicherweise verwendeten Natriumsalze für die Herstellung der Metallkomplexe von Azoverbindungen, insbesondere der Azobarbitursäure sowie deren Interkalationsverbindungen besonders vorteilhaft. Die erfindungsgemäßen Kaliumsalze können auch als Hydrate vorliegen. Das heisst, dass sie in ihrer kristallisierten Form Kristallwasser eingelagert besitzen. Bevorzugt enthalten die erfindungsgemäße Monokaliumsalze ca. 0,5 bis 1 Moläquivalent Kristallwasser.

Insbesonders bevorzugt sind Monokaliumsalze der Azobarbitursäure der Formel (IIa) als Monohydrat.

Dabei ist insbesondere jenes Monohydrat bevorzugt, das unter den Bedingungen der Differential-Scanning Calorimetry (DSC) bei einer Aufheizrate von 10 K/min bei 135°C ± 10°C ein Moläquivalent Kristallwasser abspaltet.

Ebenfalls bevorzugt ist jenes Monohydrat der Azobarbitursäure, das unter den Bedingungen der Differential-Scanning Calorimetry (DSC) bei einer Aufheizrate von 10 K/min bei 230°C ± 10°C ein Moläquivalent Kristallwasser abspaltet.

Zur Vereinfachung wird im Folgenden das Monohydrat, das sein Kristallwasser bei ca. 135°C verliert, als α-Form bezeichnet und das Monohydrat, welches sein Kristallwasser bei ca. 230°C verliert, als β-Form bezeichnet. Die Röntgenbeugungsspektren beider Hydratmodifikationen zeigen keine Unterschiede.

Weiterhin bevorzugt ist das Monokaliumsalz der Azobarbitursäure mit einem Kristallwassergehalt von 0,4 bis 0,6 Moläquivalenten, insbesondere mit ca. 0,5 Moläquivalenten Kristallwasser, das unter den Bedingungen der DSC bei einer Aufheizrate von 10 K/min bei 195°C ± 10°C das Kristallwasser abspaltet.

Die Erfindung betrifft weiterhin ein Verfahren zur Herstellung der erfindungsgemäßen Mono- oder Dikaliumsalze der Azoverbindungen der Formel (IIa), dadurch gekennzeichnet, dass man die Diazoverbindung der Formel (Va) oder (Vb),

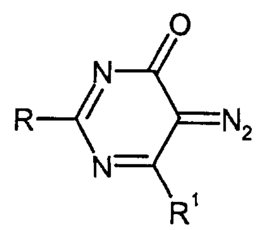
(Va),

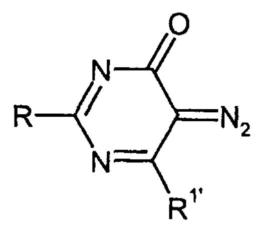
(Vb),

worin R, $R^1$, R' und $R^{1'}$ jeweils OH bedeuten
in Form ihrer freien Säure in Gegenwart von basisch reagierenden Kaliumsalzen mit Barbitursäure oder deren Derivaten der Formel (IVa) und/oder (IVb)

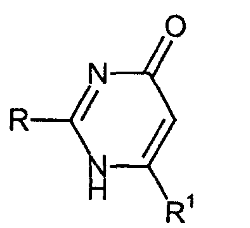
(IVa),

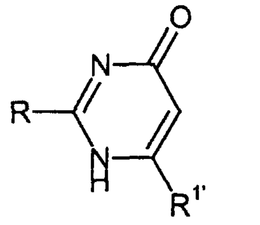
(IVb),

kuppelt.

Besonders bevorzugt ist dabei eine Ausführungsform, wobei man die Diazobarbitursäureverbindung der Formel (Va) oder (Vb) dadurch erhält, dass man ihr Na-Salz bei einem pH-Wert kleiner 1,5 in die Säure überführt und diese isoliert.

Außerdem betrifft die Erfindung weiterhin ein Verfahren zur Herstellung der erfindungsgemäßen Mono- oder Dikaliumverbindungen, das dadurch gekennzeichnet ist, dass man Barbitursäurederivate der Formel (IVa) und/oder (IVb)

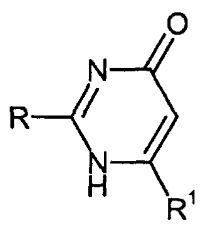

(IVa),

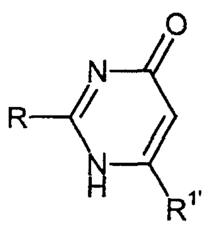

(IVb),

worin R, R', R1 und R1' die oben angegebene Bedeutung haben,
mit Azogruppenüberträgern umsetzt und das dabei erhaltene Diazobarbitursäurederivat in Gegenwart von alkalisch reagierenden Kaliumverbindungen auf Barbitursäureverbindungen der Formel (IVa) oder (IVb) kuppelt.

Besonders bevorzugt ist es, dass der Azogruppenüberträger in Gegenwart von Kaliumverbindungen, insbesondere Kaliumnitrate, als alleinige Alkaliquelle, hergestellt worden ist.

Für beide Verfahren werden als bevorzugte basisch reagierende Kaliumverbindungen KOH, $K_2CO_3$ oder Kaliumacetat eingesetzt.

Als bevorzugte Azogruppenüberträger kommen beispielsweise Umsetzungsprodukte von Aminoguanidinbicarbonat mit Kaliumnitrit in Frage, das vorzugsweise in Form seiner wässrigen Lösung zum Einsatz kommt.

Außerdem kommen Azidoverbindungen der Formel (III)

$$R^3\text{-}N_3 \qquad \text{(III)}$$

in Frage, in der

$R^3$ für einen Acylrest wie $-CONH_2$, -CO-NH-Alkyl, $-CON(Alkyl)_2$, $-COOR_{11}$, $R_{12}$-$SO_2$- oder einen heterocyclischen Rest steht. $R_{11}$ bedeutet dabei Alkyl-, Aralkyl- oder Arylreste, $R_{12}$ steht für Aminogruppen, Alkyl-, Aralkyl-, Aryl- oder heterocyclische Reste. Erfindungsgemäß werden unter Alkylresten geradkettige und verzweigtkettige, substituierte und unsubstituierte Alkylreste mit vorzugsweise 1 bis 12 C-Atomen, insbesondere mit 1 bis 6 C-Atomen, verstanden. Die Aminogruppen können beliebig substituiert sein.

Für die Synthese geeignete Azide sind beispielsweise Carbamoylazid, Azidoameisensäuremethylester, Azidoameisensäureethylester, Dimethylaminosulfonsäureazid, Methylsulfonsäureazid, Benzylsulfonsäureazid, Benzolsulfonsäureazid, o-Toluolsulfonsäureazid, m-Toluolsulfonsäureazid, p-Toluolsulfonsäureazid, 3-Nitrobenzolsulfonsäureazid, 2-Chlorbenzolsulfonsäureazid, 4-Chlorbenzolsulfonsäureazid und 2-Azido-3-ethylbenzthiazolium-tetrafluoroborat.

Bevorzugt können die Monokaliumverbindungen bei einem pH-Wert von 3 bis 7 und die Dikaliumverbindungen bei einem pH-Wert von 6 bis 9 isoliert werden.

Die erfindungsgemäßen Monokaliumsalze der Verbindungen der Formel (IIa) werden im übrigen nach beiden obengenannten Synthesewegen mit vorzugsweise 0,5 bis 1 Moläquivalent Kristallwasser erhalten. Für den Fall, dass die Monokaliumsalzhydrate getrocknet werden sollen, wird die Trocknung ihrer wässrigen Salzlösung bzw. Suspension vorzugsweise bei niedrigen Temperaturen, insbesondere bei einer Temperatur von 20 bis 60°C, insbesondere bei 40°C vorgenommen, wobei das Kristallwasser nicht abgegeben wird.

Die Erfindung betrifft daher auch ein Verfahren zur Herstellung des erfindungsgemäßen Monohydrats in der $\alpha$-Form, das dadurch gekennzeichnet ist, dass man Diazobarbitursäure in Form ihrer freien Säure in Gegenwart von basisch reagierenden Kaliumverbindungen mit Barbitursäure kuppelt und gegebenenfalls anschließend isoliert.

Behandelt man die nach diesem Verfahren erhaltene $\alpha$-Form, beispielsweise bei höheren Temperaturen unter Druck, so kann man eine weitere, ebenfalls mit einem Kristallwasser kristallisierende Form des erfmdungsgemäßen Kalziumsalzes erhalten, das der oben charakterisierten $\beta$-Form entspricht.

Die Erfindung betrifft daher auch ein Verfahren zur Herstellung des Monohydrates in der $\beta$-Form, das dadurch gekennzeichnet ist, dass man das Monohydrat in der $\alpha$-Form bei einer Temperatur von 140 bis 160°C in einem Autoklaven bei einem pH-Wert von 2 bis 6, insbesondere bei 3 bis 4 tempert.

Die beiden Modifikationen $\alpha$ und $\beta$ verhalten sich auch in der nachfolgenden Umsetzung mit entsprechenden Metallsalzen zu den Metallkomplexen der Azobarbitursäure sowie deren Interkalationsverbindungen unterschiedlich. Dies ist beispielsweise bei der Herstellung von C.I. Pigment Yellow 150 (s. Beispiele 10 und 11) zu sehen. So erhält man beispielsweise bei der Verwendung der $\alpha$-Form bei der Herstellung des entsprechenden Nickelmetallkomplexes ein Pigment, das nach der Dispergierung zu grünstichig gelben Farbtönen hoher Farbstärke führt, während ein entsprechendes Pigment, hergestellt aus der $\beta$-Form des Monohydrats zu deutlich rotstichigeren Gelbtönen geringerer Farbstärke führt. Technisch bedeutsam ist der Vorteil der Reproduzierbarkeit dieser Ergebnisse, auch verläuft die Umsetzung zu den Metallkomplexen schneller als mit den entsprechenden Na-Salzen. Dies gilt im übrigen für alle Mono- und Dikaliumsalze der Formel (IIa).

Die vorliegende Erfindung betrifft weiterhin ein Verfahren zur Herstellung von Metallkomplexen, wobei das Metall ausgewählt aus der Gruppe Ca, Zn, Cu, Fe, Cd, Sr, Ba, Cr, Sn, Al, Mg, Pd, La und Ni ist, das dadurch gekennzeichnet ist, dass man erfindungsgemäße Mono- oder Dikaliumsalze mit Metallsalzen aus der obengenannten Gruppe umsetzt, vorzugsweise bei pH $<7$, und gegebenenfalls den so entstandenen Metallkomplex mit einer einzulagernden Verbindung, vorzugsweise bei einem pH-Wert von 1 bis 7 umsetzt.

Bevorzugt weren die erfindungsgemäßen Mono- sowie Dikaliumsalze in Form ihrer wässrigen Suspensionen, vorzugsweise als wässrige Presskuchen eingesetzt.

Im allgemeinen bildet die dabei erhaltene Metallkomplexverbindung ein schichtförmiges Kristallgitter, bei dem die Bindung innerhalb einer Schicht im wesentlichen über Wasserstoffbrücken und/oder Metallionen erfolgt. Vorzugsweise handelt es sich dabei um Metallverbindungen, die ein Kristallgitter ausbilden, das aus im wesentlichen ebenen Schichten besteht.

Die Metallkomplexe mit eingelagerten anderen Verbindungen können als Einschlussverbindungen, Interkalationsverbindungen sowie als feste Lösungen vorliegen.

Als Metallkomplexe kommen auch solche in Frage, bei denen eine andere metallhaltige Verbindung, z.B. ein Salz oder Metallkomplex in das Kristallgitter des Metallkomplexes eingebaut ist. In diesem Fall kann ein Teil des Metalls im Metallkomplex durch andere Metallionen ersetzt sein oder es können weitere Metallionen in eine mehr oder weniger starke Wechselwirkung mit dem Metallkomplex treten.

Eingeschlossen sein können sowohl organische als auch anorganische Verbindungen. Verbindungen, die eingeschlossen sein können, entstammen den verschiedenartigsten Verbindungsklassen. Aus rein praktischen Gründen sind solche Verbindungen bevorzugt, die unter Normalbedingungen (25°C, 1 bar) flüssig oder fest sind.

Von den flüssigen Substanzen sind wiederum solche bevorzugt, die einen Siedepunkt (1 bar) von 100°C oder darüber, bevorzugt von 150°C und darüber, aufweisen. Geeignete Verbindungen sind vorzugsweise acyclische und cyclische organische Verbindungen, z.B. aliphatische und aromatische Kohlenwasserstoffe, die substituiert sein können, z.B. durch OH, COOH, $NH_2$, substituiertes $NH_2$, $CONH_2$, substituiertes $CONH_2$, $SO_2NH_2$, substituiertes $SO_2NH_2$, $SO_3H$, Halogen, $NO_2$, CN, $-SO_2$-Alkyl, $-SO_2$-Aryl, -O-Alkyl, -O-Aryl, -O-Acyl.

Substituenten in der Bedeutung von Aryl bezeichnen vorzugsweise Phenyl oder Naphthyl, die beispielsweise durch Halogen wie F, Cl, Br, -OH, $C_1$-$C_6$-Alkyl, $C_1$-$C_6$-Alkoxy, $-NH_2$, $-NO_2$ und -CN substituiert sein können.

Substituenten in der Bedeutung von Alkyl bezeichnen vorzugsweise $C_1$-$C_6$-Alkyl, das beispielsweise durch Halogen, wie Chlor, Brom, Fluor, -OH, -CN, $-NH_2$ oder $C_1$-$C_6$-Alkoxy substituiert sein kann.

Substituenten in der Bedeutung von Cycloalkyl bezeichnen vorzugsweise $C_3$-$C_7$-Cycloalkyl, insbesondere $C_5$-$C_6$-Cycloalkyl, das beispielsweise durch $C_1$-$C_6$-Alkyl, $C_1$-$C_6$-Alkoxy, Halogen wie Cl, Br, F, $C_1$-$C_6$-Alkoxy, -OH, -CN und $NH_2$ substituiert sein kann.

Substituenten in der Bedeutung von Aralkyl bezeichnen bevorzugt Phenyl- oder Naphthyl-$C_1$-$C_4$-alkyl, die in den aromatischen Resten beispielsweise durch Halogen wie F, Cl, Br, -OH, $C_1$-$C_6$-Alkyl, $C_1$-$C_6$-Alkoxy, $-NH_2$, $-NO_2$ und -CN substituiert sein können.

Substituenten in der Bedeutung von Acyl bezeichnen vorzugsweise ($C_1$-$C_6$-Alkyl)-carbonyl, Phenylcarbonyl, $C_1$-$C_6$-Alkylsulfonyl, Phenylsulfonyl, gegebenenfalls durch $C_1$-$C_6$-Alkyl, Phenyl und Naphthyl substituiertes Carbamoyl, gegebenenfalls durch $C_1$-$C_6$-Alkyl, Phenyl und Naphthyl substituiertes Sulfamoyl oder gegebenenfalls durch $C_1$-$C_6$-Alkyl, Phenyl und Naphthyl substituiertes Guanyl, wobei die genannten Alkylreste beispielsweise durch Halogen wie Cl, Br, F, -OH, -CN, $-NH_2$ oder $C_1$-$C_6$-Alkoxy substituiert sein können und die genannten Phenyl- und Naphthylreste beispielsweise durch Halogen wie F, Cl, Br, -OH, $C_1$-$C_6$-Alkyl, $C_1$-$C_6$-Alkoxy, $-NH_2$, $-NO_2$ und -CN substituiert sein können.

Im einzelnen seien z.B. genannt: Paraffine und Paraffinöle; Triisobutylen, Tetraisobutylen, Mischungen von aliphatischen und aromatischen Kohlenwasserstoffen, wie sie z.B. bei der Erdölfraktionierung anfallen; chlorierte Paraffinkohlenwasserstoffe wie Dodecylchlorid oder Stearylchlorid; $C_{10}$-$C_{30}$-Alkohole wie 1-Decanol, 1-Dodecanol, 1-Hexadecanol, 1-Octadecanol und ihre Mischungen, Oleinalkohol, 1,12-Octadecandiol, Fettsäuren und ihre Salze und Mischungen, z.B. Ameisensäure, Essigsäure, Dodecansäure, Hexadecansäure, Octadecansäure, Ölsäure, Fettsäureester, z.B. die Methylester der $C_{10}$-$C_{20}$-Fettsäuren, Fettsäureamide, wie Stearinsäureamid, Stearinsäuremonoethanolamid, Stearinsäurediethanolamid, Stearinsäurenitril, Fettamine, z.B. Dodecylamin, Cetylamin, Hexadecylamin, Octadecylamin und andere; Salze von Fettaminen mit Sulfon- und Carbonsäuren, isocyclische Kohlenwasserstoffe wie Cyclododecan, Decahydronaphthalin, o-, m-, p-Xylol, Mesitylen, Dodecylbenzolgemisch, Tetralin, Naphthalin, 1-Methylnaphthalin, 2-Methylnaphthalin, Biphenyl, Diphenylmethan, Acenaphthen, Fluoren, Anthracen, Phenanthren, m-, p-Terphenyl, o-, p-Dichlorbenzol, Nitrobenzol, 1-Chlomaphthalin, 2-Chlornaphthalin, 1-Nitronaphthalin, isocyclische Alkohole und Phenole und ihre Abkömmlinge wie Benzylalkohol, Decahydro-2-naphthol, Diphenylether, Sulfone, z.B. Diphenylsulfon, Methylphenylsulfon, 4,4'-Bis-2-(hydroxyethoxy)-diphenylsulfon; isocyclische Carbonsäuren und ihre Derivate wie Benzoesäure, 3-Nitrobenzoesäure, Zimtsäure, 1-Naphthalincarbonsäure, Phthalsäure, Phthalsäuredibutylester, Phthalsäuredioctylester, Tetrachlorphthalsäure, 2-Nitrobenzamid, 3-Nitrobenzamid, 4-Nitrobenzamid, 4-Chlorbenzamid, Sulfonsäuren, wie 2,5-Dichlorbenzolsulfonsäure, 3-Nitro-, 4-Nitro-benzolsulfonsäure, 2,4-Dimethylbenzolsulfonsäure, 1- und 2-Naphthalinsulfonsäure, 5-Nitro-1- und 5-Nitro-2-naphthalinsulfonsäure, Di-sec.-butyl-naphthalinsulfonsäuregemisch, Biphenyl-4-sulfonsäure, 1,4-, 1,5-, 2,6-, 2,7-Naphthalindisulfonsäure, 3-Nitro-1,5-naphthalindisulfonsäure, Anthrachinonsulfonsäure-1, Anthrachinonsulfonsäure-2, Diphenyl-4,4'-disulfonsäure, 1,3,6-Naphthalintrisulfonsäure und die Salze dieser Sulfonsäuren z.B. die Natrium-, Kalium-, Calcium-, Zink-, Nickel- und Kupfersalze; Sulfonamide wie Benzolsulfonamid, 2-, 3- und 4-Nitrobenzolsulfonamid, 2-, 3- und 4-Chlorbenzolsulfonamid, 4-Methoxy-benzolsulfonamid, 3,3'-Sulfonylbisbenzolsulfonamid, 4,4'-Oxybisbenzolsulfonsäureamid, 1- und 2-Naphthalinsulfonsäureamid.

Carbonsäure- und Sulfonsäureamide sind eine bevorzugte Gruppe von einzuschließenden Verbindungen, insbesondere geeignet sind auch Harnstoff und substituierte Harnstoffe wie Phenylharnstoff, Dodecylharnstoff und andere, sowie deren Polykondensate mit Aldehyden, insbesondere Formaldehyd; Heterocyclen wie Barbitursäure, Benzimidazolon, Benzimidazolon-5-sulfonsäure, 2,3-Dihydroxychinoxalin, 2,3-Dihydroxychinoxalin-6-sulfonsäure, Carbazol, Carbazol-3,6-disulfonsäure, 2-Hydroxychinolin, 2,4-Dihydroxychinolin, Caprolactam, Melamin, 6-Phenyl-1,3,5-triazin-2,4-diamin, 6-Methyl-1,3,5-triazin-2,4-diamin und Cyanursäure.

Bevorzugte Metallkomplexe enthalten eingeschlossen grenzflächenaktive Verbindungen, insbesondere Tenside, die z.B. aus K. Lindner, Tenside-Textilhilfsmittel-Waschrohstoffe, 2. Auflage, Band I, Wissenschaftliche Verlagsgesellschaft mbH, Stuttgart, 1964, bekannt sind. Dabei kann es sich um anionenaktive, nichtionogene oder kationenaktive Verbindungen oder um Ampholyte handeln. Geeignete anionenaktive Verbindungen sind z.B.: Echte Seifen, Salze der Aminocarbonsäuren, Salze niederer bzw. höherer acylierter Aminocarbonsäuren, Fettsäuresulfate, Sulfate von Fettsäureestern, -amiden usw., primäre Alkylsulfate, Sulfate von Oxoalkoholen, sekundäre Alkylsulfate, Sulfate veresterter oder veretherter Polyoxyverbindungen, Sulfate substituierter Polyglykolether (sulfatierte Ethylenoxidaddukte), Sulfate acylierter oder alkylierter Alkanolamine, Sulfonate von Fettsäuren, ihren Estern, Amiden usw., primäre Alkylsulfonate, sekundäre Alkylsulfonate, Alkylsulfonate mit esterartig gebundenen Acylen, Alkyl- bzw. Alkylphenylethersulfonate, Sulfonate von Polycarbonsäureestern, Alkylbenzolsulfonate, Alkylnaphthalinsulfonate, fettaromatische Sulfonate, Alkylbenzimidazolsulfonate, Phosphate, Polyphosphate, Phosphonate, Phosphinate, Thiosulfate, Hydrosulfite, Sulfinate, Persulfate. Geeignete nichtionogene Verbindungen sind z.B.: Ester und Ether von Polyalkoholen, Alkylpolyglykolether, Acylpolyglykolether, Alkylarylpolyglykolether, acylierte bzw. alkylierte Alkanolaminpolyglykolether. Geeignete kationenaktive Verbindungen sind z.B.: Alkylaminsalze, quaternäre Ammoniumsalze, Alkylpyridiniumsalze, einfache und quaternäre Imidazolinsalze, Alkyldiamine bzw. Alkylpolyamine, Acyldiamine bzw. Acylpolyamine, Acylalkanolamine, Alkanolaminester, Alkyl-$OCH_2$-N-pyridiniumsalze, Alkyl-CO-NH-$CH_2$-N-pyridiniumsalze, Alkylethylenharnstoffe, Sulfoniumverbindungen, Phosphoniumverbindungen, Arseniumverbindungen, Alkylguanidine, Acylbiguanidide. Geeignete Ampholyte sind z.B.: Alkylbetaine, Sulfobetaine und Aminocarbonsäuren. Bevorzugt eingesetzt werden nichtionogene Tenside, insbesondere die Ethylenoxidadditionsprodukte von Fettalkoholen, Fettaminen sowie von Octyl- oder Nonylphenol.

Eine weitere wichtige Gruppe von eingelagerten Verbindungen sind natürliche Harze und Harzsäuren wie z. B. Abietinsäure und ihre Umwandlungsprodukte und Salze.

Solche Umwandlungsprodukte sind z.B. hydrierte, dehydrierte und disproportionierte Abietinsäuren. Diese können weiterhin dimerisiert, polymerisiert oder durch Addition von Maleinsäureanhydrid und Fumarsäure modifiziert sein. Von Interesse sind auch die an der Carboxylgruppe modifizierten Harzsäuren wie z.B. die Methyl-, Hydroxyethyl-, Glykol-, Glycerin- und Pentaerythritester, sowie Harzsäurenitrile und Harzsäureamine sowie Dehydroabietylalkohol.

Ebenfalls für eine Einlagerung geeignet sind Polymere, vorzugsweise wasserlösliche Polymere, z.B. Ethylenpropylenoxid-Blockcopolymere, vorzugsweise mit einem MG größer gleich 1 000, insbesondere von 1 000 bis 10 000 g/mol, Polyvinylalkohol, Poly-(meth)-acrylsäuren, modifizierte Cellulose, wie Carboxymethylcellulosen, Hydroxyethyl- und -propylcellulosen, Methyl- und Ethylhydroxyethylcellulosen.

Ebenfalls für die Einlagerung geeignet sind Kondensationsprodukte auf Basis von

A) sulfonierten Aromaten,

B) Aldehyden und/oder Ketonen und gegebenenfalls

C) einer oder mehrerer Verbindungen, ausgewählt aus der Gruppe der nicht sulfonierten Aromaten, Harnstoff und Harnstoffderivaten.

Auf Basis von bedeutet, dass das Kondensationsprodukt gegebenenfalls aus weiteren Reaktanden neben A, B und gegebenenfalls C hergestellt wurde. Vorzugsweise werden die Kondensationsprodukte im Rahmen dieser Anmeldung jedoch nur aus A, B und gegebenenfalls C hergestellt.

Als sulfonierte Aromaten der Komponente A) werden im Rahmen dieser Anmeldung auch sulfomethylierte Aromaten verstanden. Bevorzugte sulfonierte Aromaten sind: Naphthalinsulfonsäuren, Phenolsulfonsäuren, Dihydroxybenzolsulfonsäuren, sulfonierte Ditolylether, sulfomethyliertes 4,4'-Dihydroxydiphenylsulfon, sulfoniertes Diphenylmethan, sulfoniertes Biphenyl, sulfoniertes Hydroxybiphenyl, insbesondere 2-Hydroxybiphenyl, sulfoniertes Terphenyl oder Benzolsulfonsäuren.

Als Aldehyde und/oder Ketone der Komponente B) kommen insbesondere aliphatische, cycloaliphatische sowie aromatische in Frage. Bevorzugt sind aliphatische Aldehyde, wobei besonders bevorzugt Formaldehyd sowie andere aliphatische Aldehyde mit 3 bis 5 C-Atomen in Frage kommen.

Als nicht sulfonierte Aromaten der Komponente C) kommen beispielsweise Phenol, Kresol, 4,4'-Dihydroxydiphenylsulfon oder Dihydroxydiphenylmethan in Frage.

Als Harnstoffderivate können beispielsweise Dimethylolharnstoff, Alkylharnstoffe, Melamin oder Guanidin genannt werden.

Als bevorzugtes Kondensationsprodukt wird eines auf Basis von

A) wenigstens einem sulfonierten Aromaten, ausgewählt aus der Gruppe von Naphthalinsulfonsäuren, Phenolsulfonsäuren, Dihydroxybenzolsulfonsäuren, sulfonierte Ditolylether, sulfomethyliertes 4,4'-Dihydroxydiphenylsulfon, sulfoniertes Diphenylmethan, sulfoniertes Biphenyl, sulfoniertes Hydroxybiphenyl, insbesondere 2-Hydroxybiphenyl, sulfoniertes Terphenyl und Benzolsulfonsäuren,

B) Formaldehyd und gegebenenfalls

C) einer oder mehreren Verbindungen, ausgewählt aus der Gruppe von Phenol, Kresol, 4,4'-Dihydroxydiphenylsulfon, Dihydroxydiphenylmethan, Harnstoff, Dimethylolharnstoff, Melamin und Guanidin

eingesetzt.

Bevorzugte Kondensationsprodukte sind solche auf Basis von 4,4'-Dihydroxydiphenylsulfon, sulfonierter Ditolylether und Formaldehyd; 4,4'-Dihydroxydiphenylsulfon, Phenolsulfonsäure und Formaldehyd; 4,4'-Dihydroxydiphenylsulfon, Natriumbisulfit, Formaldehyd und Harnstoff; Naphthalinsulfonsäure, 4,4'-Dihydroxydiphenylsulfon und Formaldehyd; sulfoniertes Terphenyl und Formaldehyd; und/oder sulfoniertes 2-Hydroxybiphenyl und Formaldehyd sowie Naphthalinsulfonsäure und Formaldehyd.

Besonders bevorzugt werden als eingelagerte Verbindungen, Melamin oder Melaminderivat, insbesondere solche der Formel (IV)

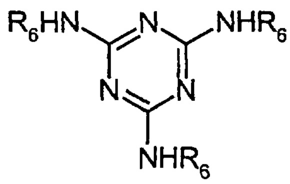

(IV)

eingesetzt, worin

$R_6$ für Wasserstoff oder $C_1$-$C_4$-Alkyl, das gegebenenfalls mit OH-Gruppen substituiert ist, steht,

ganz besonders bevorzugt, worin

$R_6$ für Wasserstoff steht.

Die Menge an Substanz, die in das Kristallgitter des Metallkomplexes eingelagert werden kann, liegt in der Regel bei 5 % bis 200 Gew.-%, bezogen auf die Menge an Wirtsverbindung. Bevorzugt eingelagert werden 10 bis 100 Gew.-%. Es handelt sich hierbei um die Menge an Substanz, die durch geeignete Lösungsmittel nicht auswaschbar ist und die sich aus der Elementaranalyse ergibt. Naturgemäß kann auch mehr oder weniger als die genannte Menge an Substanz zugesetzt werden, wobei man gegebenenfalls darauf verzichten kann, einen Überschuss auszuwaschen. Bevorzugt sind Mengen von 10 bis 150 Gew.-%.

Einschlussverbindungen, Interkalationsverbindungen und feste Lösungen von Metallkomplexen an sich sind aus der Literatur bekannt. Sie werden ebenso wie deren Herstellung beispielsweise in der EP 0 074 515 und der EP 0 073 463 beschrieben.

Eine bevorzugte Ausführungsform des erfindungsgemäßen Verfahrens zur Herstellung der Metallkomplexe ist dadurch gekennzeichnet, dass im Anschluss an die Einlagerung der pH auf größer gleich 4,5 vorzugsweise auf 4,5 bis 7 angehoben wird, sofern die Einlagerung selbst bei einem pH von kleiner 4,5 erfolgte.

Als Metallsalz kommen vorzugsweise wasserlösliche Metallsalze der oben genannten Metalle in Frage, insbesondere Chloride, Bromide, Acetate, Nitrate usw. Bevorzugt eingesetzte Metallsalze besitzen eine Wasserlöslichkeit von mehr als 20 g/l, insbesondere mehr als 50 g/l bei 20°C.

Geeignete Metallsalze zur Herstellung der Salze und Komplexe der Azoverbindungen sind beispielsweise: Magnesiumchlorid, Magnesiumsulfat, Calciumchlorid, Calciumacetat, Calciumformiat, Bariumchlorid, Bariumnitrat, Bariumacetat, Bariumcarbonat, Strontiumnitrat, Manganchlorid, Mangansulfat, Eisen-(III)-chlorid, Eisen-(III)-nitrat, Eisen-(II)-sulfat, Cobaltchlorid, Cobaltnitrat, Cobaltsulfat, Nickelnitrat, Nickelsulfat, Nichelchlorid, Nickelacetat, Nickelformiat, Aluminiumsulfat, Aluminiumnitrat, Chrom-(III)-sulfat, Chrom-(III)-nitrat, Zinkchlorid, Zinksulfat, Zinkacetat, Cadmiumchlorid, Cadmiumsulfat, Cadmiumnitrat, Kupfer-(II)-sulfat, Kupfer-(II)-chlorid, Kupfer-(II)-acetat und Kupfer-(II)-formiat, Lanthannitrat und Aluminiumchloridhydrat.

Man kann auch Mischungen dieser Salze, die verschiedene der genannten Metalle enthalten können, verwenden. Die Verwendung von solchen Salzmischungen empfiehlt sich insbesondere für die Erzielung von Zwischentönen der farbigen Endprodukte.

Die Erfindung betrifft weiterhin Kaliummetallkomplexe der erfindungsgemäßen Mono- oder Dikaliumsalze der Azoverbindungen der Formel (IIa), die wenigstens eine Verbindung eingelagert enthalten.

Als eingelagerte Verbindungen sind die oben genannten anorganischen und vorzugsweise organischen Verbindungen zu nennen.

Unter Metallkomplexen werden im Rahmen dieser Anmeldung auch Metallsalze verstanden.

Die Erfindung betrifft weiterhin ein Verfahren zur Herstellung der erfindungsgemäßen Kaliummetallkomplexe, das dadurch gekennzeichnet ist, dass man die erfindungsgemäßen Mono- oder Dikaliumsalze der Azoverbindung der Formel (IIa) mit der einzulagernden Verbindung umsetzt, vorzugsweise bei einem pH-Wert von 1 bis 7.

Als besonders bevorzugte einzulagernde Verbindungen sind zu nennen: cyclische oder acyclische oder organische Verbindungen, wie sie bereits oben beschrieben sind, insbesondere Melamin oder Melaminderivate oder Polykondensate, bevorzugt solche auf Basis von Harnstoff und Formaldehyd sowie Ethylenoxidpropylenoxid Block Copolymerisate.

Die erfindungsgemäßen Kaliummetallkomplexe, die eine andere Verbindung eingelagert enthalten, eignen sich hervorragend für alle Pigmentanwendungszwecke. Zum Beispiel eignen sie sich zum Pigmentieren von Lacken aller Art für die Herstellung von Druckfarben, Leimfarben oder Binderfarben, für die Massefärbung von synthetischen, halbsynthetischen oder natürlichen makromolekularen Stoffen, wie z.B. Polyvinylchlorid, Polystyrol, Polyamid, Polyethylen oder Polypropylen. Sie können auch für die Spinnfärbung von natürlichen, regenerierten oder künstlichen Fasern, wie z.B. Cellulose-, Polyester-, Polycarbonat-, Polyacrylnitril- oder Polyamidfasern, sowie zum Bedrucken von Textilien und Papier verwendet werden. Aus diesen Pigmenten können feinteilige, stabile, wässrige Pigmentierung von Dispersions- und Anstrichfarben, für die Papierfärbung, für den Pigmentdruck von Textilien, für den Laminatdruck oder für die Spinnfärbung von Viskose brauchbar sind, durch Mahlen oder Kneten in Gegenwart von nichtionogenen, anionischen oder kationischen Tensiden hergestellt werden.

**Beispiele**

**Beispiel 1:** α-Modifikation Azobarbitursäure Monokaliumsalz x 1 $H_2O$

In 810 g dest. Wasser werden 136 g Aminoguanidinbicarbonat eingetragen und mit 280 g Salzsäure (30 %ig ) gelöst. Danach wird mit 780g Eis auf ca. -10°C abgekühlt und anschließend mit 232 g einer 37 %igen Kaliumnitritlösung in Wasser bis ca. 15°C versetzt. Danach wird bei ca. 15°C 15 Min. nachgerührt und anschließend mit 2,0 g Amidoschwefelsäure versetzt. Danach werden 269 g Barbitursäure eingetragen, anschließend wird auf 55°C erwärmt und 2 Std. nachgerührt. Anschließend wird mit wässriger Kalilauge auf pH 2,5 eingestellt und 30 Min. nachgerührt. Danach wird mit wässriger Kalilauge auf pH 4,8 eingestellt und 30 Min. nachgerührt. Anschließend wird der Ansatz auf 80°C erwärmt und 3 Std. bei pH 4,8 nachgerührt. Danach wird auf einer Saugnutsche isoliert, elektrolytfrei gewaschen, bei 40°C im Umlufttrockenschrank getrocknet und gemahlen. Man erhält 334 g eines orangenroten Pulvers.

| | | | | |
|---|---|---|---|---|
| Berechnung für Monokaliumsalz x 1 $H_2O$ | C 28,4 | H 2,1 | N 24,8 | K 11,6 |
| Gefunden | C 27,8 | H 2,4 | N 24,3 | K 12,0 |

Die Differential Scanning Calorimetry (DSC) ist eine spezielle Form der Differentialthermoanalyse. Die DSC-Analysen wurden auf einen Gerät der Firma Mettler in einem Ofen des Typs DSC 20 gemessen. Die Proben wurden im Tiegel mit dreifach gelochten Deckel bei einer Aufheizrate von 10 K/Minute untersucht.

Unter den genannten Bedingungen gibt die nach der obigen Vorschrift hergestellte Verbindung ein endothermes Signal bei 135°C. (Die genannte Temperatur von 135°C kann , bedingt durch die Reproduzierbarkeitgrenzen der DSC-Methode um ± 10°C schwanken.) Aus der Differentialthermoanalyse in Verbindung mit Fourier-Transform-IR-Analyse folgt, dass bei der genannten Temperatur ein Moläquivalent Wasser abgegeben wird.

**Beispiel 2:** α-Modifikation Azobarbitursäure Monokaliumsalz x 1 $H_2O$

Man verfährt wie in Beispiel 1 beschrieben, setzt jedoch anstelle von Kalilauge Kaliumacetatlösung ein. Die so erhaltene Verbindung wird wie in Beispiel 1 beschrieben isoliert und getrocknet.

Nach DSC-Analyse ist die Verbindung identisch mit der nach Beispiel 1 hergestellten Verbindung.

**Beispiel 3:** α-Modifikation Azobarbitursäure Monokaliumsalz x 1 $H_2O$

Man verfährt wie in Beispiel 1 beschrieben, setzt jedoch anstelle von Kalilauge Kaliumcarbonatlösung ein. Die so erhaltene Verbindung wird wie in Beispiel 1 beschrieben isoliert und getrocknet.

Nach DSC-Analyse ist die Verbindung identisch mit der nach Beispiel 1 hergestellten Verbindung.

**Beispiel 4:** α-Modifikation Azobarbitursäure Monokaliumsalz x 1 $H_2O$

In 1 850 g dest. Wasser werden 154 g Diazobarbitursäure und 131 g Barbitursäure eingetragen, dann wird auf 80°C erwärmt und 30 Min. nachgerührt. Anschließend wird mit wässriger Kalilauge auf pH 4,8 gestellt und 3 Std. nachgerührt. Danach wird auf einer Saugnutsche isoliert, elektrolytfrei gewaschen und bei 40°C im Umlufttrockenschrank getrocknet und gemahlen. Man erhält 334 g eines orangenroten Pulvers.

Nach DSC-Analyse ist die Verbindung identisch mit der nach Beispiel 1 hergestellten Verbindung.

| | | | | |
|---|---|---|---|---|
| Berechnung für Monokaliumsalz x 1 $H_2O$ | C 28,4 | H 2,1 | N 24,8 | K 11,6 |
| Gefunden | C 27,5 | H 2,4 | N 24,3 | K 11,0 |

**Beispiel 5:** α-Modifikation Azobarbitursäure Monokaliumsalz x 1 $H_2O$

Man verfährt wie in Beispiel 4 beschrieben, setzt jedoch anstelle von Kalilauge Kaliumacetatlösung ein. Die

so erhaltene Verbindung wird wie in Beispiel 1 beschrieben isoliert und getrocknet.

Nach DSC-Analyse ist die Verbindung identisch mit der nach Beispiel 1 hergestellten Verbindung.

**Beispiel 6:** α-Modifikation Azobarbitursäure Monokaliumsalz x 1 $H_2O$

Man verfährt wie in Beispiel 4 beschrieben, setzt jedoch anstelle von Kalilauge Kaliumcarbonatlösung ein. Die so erhaltene Verbindung wird wie in Beispiel 1 beschrieben isoliert und getrocknet.

Nach DSC-Analyse ist die Verbindung identisch mit der nach Beispiel 1 hergestellten Verbindung.

**Beispiel 7:** β-Modifikation Azobarbitursäure Monokaliumsalz x 1 $H_2O$

In 2 700 g dest. Wasser werden 300 g α-Modifikation Azobarbitursäure Monokaliumsalz x 1 $H_2O$ hergestellt nach Beispiel 1, mit einen Laborrührer homogen verrührt. Danach wird mit Salzsäure ein pH von 3,0 eingestellt und im Autoklaven 8 Std. bei 140°C nachgerührt. Anschließend wird auf 80°C abgekühlt, auf einer Saugnutsche isoliert, elektrolytfrei gewaschen, bei 40°C im Umlufttrockenschrank getrocknet und gemahlen. Man erhält 240 g eines orangeroten Pulvers.

| | | | | |
|---|---|---|---|---|
| Berechnung für Monokaliumsalz x 1 $H_2O$ | C 28,4 | H 2,1 | N 24,8 | K 11,6 |
| Gefunden | C 28,4 | H 2,2 | N 24,8 | K 11,0 |

Bei der DSC-Analyse gibt die nach der obigen Vorschrift hergestellte Verbindung ein endothermes Signal bei 230°C ± 10°C. Aus der Differentialthermoanalyse in Verbindung mit Fourier-Transform-IR-Analyse folgt, dass bei der genannten Temperatur ein Moläquivalent Wasser abgegeben wird.

**Beispiel 8:** Azobarbitursäure Monokaliumsalz x ½ $H_2O$

Man verfährt wie in Beispiel 1 beschrieben, trocknet jedoch im Vakumtrockenschrank bei 120°C. Man erhält 316 g eines orangeroten Pulvers.

| | | | | |
|---|---|---|---|---|
| Berechnung für Monokaliumsalz x ½ $H_2O$ | C 30,0 | H 1,6 | N 26,2 | K 12,2 |
| Gefunden | C 29,2 | H 1,9 | N 25,0 | K 12,1 |

Bei der DSC-Analyse gibt die nach der obigen Vorschrift hergestellte Verbindung ein endothermes Signal bei 195°C+- 10°C. Aus der Differentialthermoanalyse in Verbindung mit Fourier-Transform-IR-Analyse folgt, dass bei der genannten Temperatur ein ½ Moläquivalent Wasser abgegeben wird.

**Beispiel 9:** Azobarbitursäure Monokaliumsalz x ½ $H_2O$

Man verfährt wie in Beispiel 4 beschrieben, trocknet jedoch im Vakumtrockenschrank bei 120°C. Man erhält 316 g eines orangeroten Pulvers.

Nach DSC-Analyse ist die Verbindung identisch mit der nach Beispiel 8 hergestellten Verbindung.

| | | | | |
|---|---|---|---|---|
| Berechnung für Monokaliumsalz x ½ $H_2O$ | C 30,0 | H 1,6 | N 26,2 | K 12,2 |
| Gefunden | C 29,2 | H 1,9 | N 25,3 | K 12,1 |

**Beispiel 10:** Mit Melamin interkalierter Ni-Komplex der Azobarbitursäure

425 g wasserfeuchte Paste der nach Beispiel 1 hergestellte α-Modifikation des Azobarbitursäure Monokaliumsalz *H2O mit einem Trockengehalt von 40 %, entsprechend 170 g trocken, werden in 5 000 ml destilliertem Wasser mit einem Laborrührer homogen verrührt, auf 95°C erwärmt mit 126g Melamin versetzt. Anschließend werden 1 060 g wässrige 6,5 %ige Nickelchloridlösung zugesetzt und 1,5 Std. bei 95°C nachgerührt. Danach wird mit Kalilauge auf pH 5,5 gestellt. Anschließend wird auf einer Saugnutsche isoliert, elektrolytfrei gewaschen, bei 80°C im Vakuumtrokkenschrank getrocknet und gemahlen. Man erhält 288 g eines grünlichen gelben Pulvers.

Das so erhaltene Pigmentpulver wird in ein Weißlackaufstrich beurteilt. Dafür wird das Pigment entsprechend den Angaben von DIN 53 238 Teil 31 in einen Alkyd-Melaminharzsystem eingearbeitet.

Farbort: x = 0,4350 y = 0,4671 mit

$$x = \frac{X}{X+Y+Z} \qquad y = \frac{Y}{X+Y+Z}$$

wobei
X, Y und Z Normfarbwerte sind.
(Das Normfarbsystem ist beschrieben in Bayer Farben Revue, Sonderheft 3/2 D, 1986; S. 12-14).

**Beispiel 11:** Mit Melamin interkalierter Ni-Komplex der Azobarbitursäure

Man verfährt wie in Beispiel 10 beschrieben, setzt jedoch die nach Beispiel 7 hergestellte wasserfeuchte Paste der β-Modifikation der Azobarbitursäure Monokaliumsalz x $H_2O$ ein. Man erhält 288 g eines gelblichen Pulvers.
Das so erhaltene Pigmentpulver wird in ein Weißlackaufstrich beurteilt. Dafür wird das Pigment entsprechend den Angaben von DIN 53238 Teil 31 in einen Alkyd-Melaminharzsystem eingearbeitet.

Farbort: x = 0,4406 y = 0,4624 Farbstärke: 71 % der Farbstärke des nach Beispiel 10 erhaltenen Pigments.

**Beispiel 12:** Azobarbitursäure Dikaliumsalz

In 1 850 g dest. Wasser werden 154 g Diazobarbitursäure und 131 g Barbitursäure eingetragen, auf 80°C erwärmt und 30 Min. nachgerührt. Anschließend wird mit wässriger Kalilauge auf pH 4,8 eingestellt und 3 Std. nachgerührt. Anschließend wird mit wässriger Kalilauge auf pH 10,0 gestellt und 3 Std. nachgerührt. Danach wird auf einer Saugnutsche isoliert, elektrolytfrei gewaschen und bei 120°C im Vakumtrockenschrank getrocknet. Man erhält 341 g eines orangeroten Pulvers.

| | | | | |
|---|---|---|---|---|
| Berechnet für Dikaliumsalz | C 26,7 | H 1,4 | N 23,4 | K 21,8 |
| Gefunden | C 26,2 | H 1,6 | N 22,9 | K 21,0 |

**Patentansprüche**

1. Mono- oder Dikaliumsalze der Azobarbitursäure der Formel (IIa)

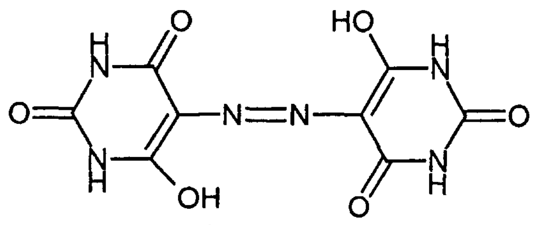

(IIa)

sowie ihre Hydrate.

2. Monokaliumsalz der Azobarbitursäure gemäß Anspruch 1 sowie seine Hydrate.

3. Monokaliumsalz der Azobarbitursäure gemäß Anspruch 2 als Monohydrat.

4. Monokaliumsalz gemäß Anspruch 2, **dadurch gekennzeichnet, dass** das Monohydrat unter den Bedingungen der Differential-Scanning Calorimetry (DSC) bei einer Aufheizrate von 10 K/min bei einer Temperatur von 135°C ± 10°C ein Moläquivalent Kristallwasser abspaltet.

5. Monokaliumsalz gemäß Anspruch 2, **dadurch gekennzeichnet, dass** das Monohydrat unter den Bedingungen der Differential-Scanning Calorimetry (DSC) bei einer Aufheizrate von 10 K/min bei einer Temperatur von 230°C ± 10°C ein Moläquivalent Kristallwasser abspaltet.

6. Monokaliumsalz gemäß Anspruch 2, **dadurch gekennzeichnet, dass** das Hydrat unter den Bedingungen der DSC bei einer Aufheizrate von 10 K/min bei einer Temperatur von 195°C ± 10°C 0,4 bis 0,6, vorzugsweise 0,5 Moläquivalente Kristallwasser abspaltet.

7. Verfahren zur Herstellung von Mono- oder Dikaliumsalzen gemäß Anspruch 1, **dadurch gekennzeichnet, dass** man die Diazoverbindung der Formel (Va) oder (Vb),

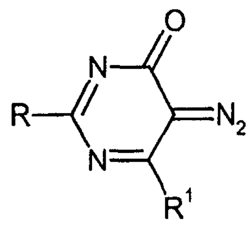

(Va),

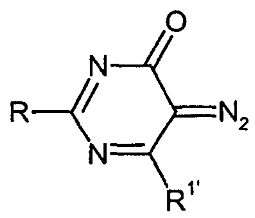

(Vb),

worin R, $R^1$, R' und $R^{1'}$ jeweils OH bedeuten, in Form ihrer freien Säure in Gegenwart von basisch reagierenden Kaliumsalzen mit Barbitursäure oder deren Derivaten der Formel (IVa) und/oder (IVb)

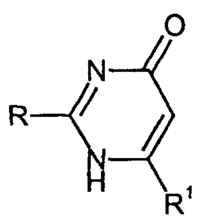

(IVa),

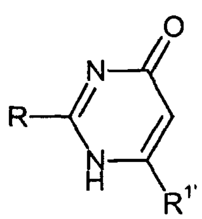

(IVb),

kuppelt.

8. Verfahren nach Anspruch 7, **dadurch gekennzeichnet, dass** man die Diazobarbitursäureverbindung dadurch erhält, dass man ihr Na-Salz bei einem pH-Wert kleiner 1,5 in die Säure überführt und diese isoliert.

9. Verfahren zur Herstellung von Mono- oder Dikaliumverbindungen gemäß Anspruch 1, **dadurch gekennzeichnet, dass** man Barbitursäurederivate der Formel (IVa) und/oder (IVb)

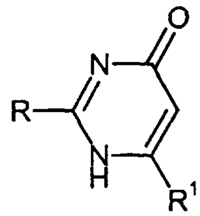

(IVa),

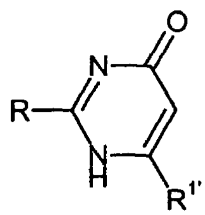

(IVb),

worin R, R', $R^1$ und $R^{1'}$ jeweils OH bedeuten mit Azogruppenüberträgern umsetzt und das dabei erhaltene Diazobarbitursäurederivat in Gegenwart von alkalisch reagierenden Kaliumverbindungen auf Barbitursäureverbindungen der Formel (IVa) oder (IVb) kuppelt.

**10.** Verfahren gemäß Anspruch 9, **dadurch gekennzeichnet, dass** der Azogruppenüberträger in Gegenwart von Kaliumverbindungen, insbesondere Kaliumnitrit als alleinige Alkaliquelle hergestellt worden ist.

**11.** Kaliummetallkomplexe einer Azo-Verbindung gemäß Anspruch 1, die wenigstens eine Verbindung eingelagert enthalten.

**12.** Verfahren zur Herstellung der Kaliummetallkomplexe gemäß Anspruch 11, **dadurch gekennzeichnet, dass** man das Mono- und/oder Dikaliumsalz gemäß Anspruch 1 mit einer einzulagernden Verbindung, insbesondere einer organischen Verbindung umsetzt, insbesondere bei einem pH-Wert von 1 bis 7.

**13.** Verfahren zur Herstellung von Metallkomplexen, wobei die Metalle ausgewählt sind aus der Gruppe Ca, Zn, Cu, Fe, Cd, Sr, Ba, Cr, Sn, Al, Mg, Pd, La und Ni, **dadurch gekennzeichnet, dass** man die Kaliumsalze gemäß Anspruch 1 vorzugsweise bei pH <7 und gegebenenfalls den entstandenen Metallkomplex mit einer einzulagernden Verbindung vorzugsweise bei pH-Werten von 1 bis 7 umsetzt.

**14.** Verfahren zur Herstellung des Monohydrats gemäß Anspruch 4, **dadurch gekennzeichnet, dass** man Diazobarbitursäure in Form ihrer freien Säure in Gegenwart von basisch reagierenden Kaliumverbindungen mit Barbitursäure kuppelt und gegebenenfalls anschließend isoliert.

**15.** Verfahren zur Herstellung des Monohydrats gemäß Anspruch 5, **dadurch gekennzeichnet, dass** man das Monohydrat nach Anspruch 4 bei einer Temperatur von 140 bis 160°C im Autoklaven bei einem pH-Wert von 2 bis 6 tempert.

## Claims

**1.** Mono- or dipotassium salts of azobarbituric acid of the formula (IIa)

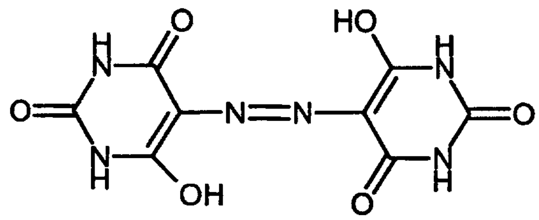

(IIa)

and also their hydrates.

2. Monopotassium salt of azobarbituric acid according to Claim 1 and also its hydrates.

3. Monopotassium salt of azobarbituric acid according to Claim 2 as monohydrate.

4. Monopotassium salt according to Claim 2, **characterized in that** the monohydrate detaches one mole equivalent of water of crystallization at a temperature of 135°C ± 10°C when subjected to the conditions of differential scanning calorimetry (DSC) with a heating rate of 10 K/min.

5. Monopotassium salt according to Claim 2, **characterized in that** the monohydrate detaches one mole equivalent of water of crystallization at a temperature of 230°C ± 10°C when subjected to the conditions of differential scanning calorimetry (DSC) with a heating rate of 10 K/min.

6. Monopotassium salt according to Claim 2, **characterized in that** the hydrate detaches 0.4 to 0.6, preferably 0.5, mol equivalent of water of crystallization at a temperature of 195°C ± 10°C when subjected to the conditions of DSC with a heating rate of 10 K/min.

7. Process for preparing mono- or dipotassium salts according to Claim 1, **characterized in that** the diazo compound of the formula (Va) or (Vb)

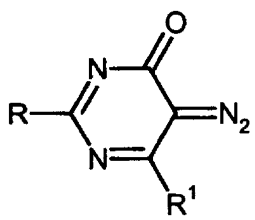

(Va),

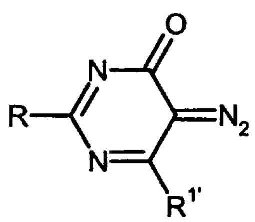

(Vb),

where R, $R^1$, R' and $R^{1'}$ are each OH, in the form of its free acid is coupled in the presence of alkaline potassium salts with barbituric acid or its derivatives of the formula (IVa) and/or (IVb)

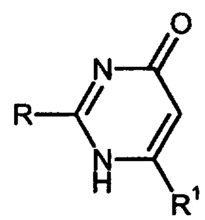

(IVa),

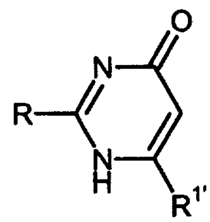

(IVb).

8. Process according to Claim 7, **characterized in that** the diazobarbituric acid compound is obtained by converting its sodium salt to the acid at a pH of less than 1.5 and isolating the acid.

9. Process for preparing mono- or dipotassium compounds according to Claim 1, **characterized in that** barbituric acid derivatives of the formula (IVa) and/or (IVb)

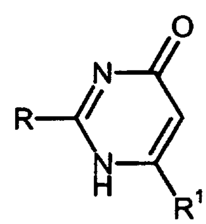

(IVa),

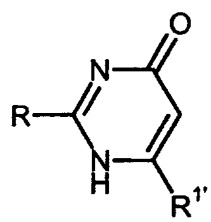

(IVb),

where R, R', $R^1$ and $R^{1'}$ are each OH, are reacted with azo group transfer agents and the resulting diazobarbituric acid derivative is coupled onto barbituric acid compounds of the formula (IVa) or (IVb) in the presence of alkaline potassium compounds.

10. Process according to Claim 9, **characterized in that** the azo group transfer agent is prepared in the presence of potassium compounds, especially potassium nitrite, as sole alkali source.

11. Potassium metal complexes of an azo compound according to Claim 1 which contains at least one guest compound.

12. Process for preparing potassium metal complexes of Claim 11, **characterized in that** the mono- and/or dipotassium salt of Claim 1 is reacted with a compound to be hosted, especially an organic compound, especially at a pH of 1 to 7.

13. Process for preparing metal complexes where the metals are selected from the group consisting of Ca, Zn, Cu,

Fe, Cd, Sr, Ba, Cr, Sn, Al, Mg, Pd, La and Ni, **characterized in that** the potassium salts of Claim 1 are preferably reacted at pH <7 and, if desired, the resulting metal complex is preferably reacted with a compound to be hosted at pH 1 to 7.

**14.** Process for preparing the monohydrate of Claim 4, **characterized in that** diazobarbituric acid in the form of its free acid is coupled with barbituric acid in the presence of alkaline potassium compounds and if desired the product subsequently isolated.

**15.** Process for preparing the monohydrate of Claim 5, **characterized in that** the monohydrate of Claim 4 is heat-treated at a temperature of 140 to 160°C in an autoclave at a pH of 2 to 6.

**Revendications**

**1.** Sels mono- ou di-potassiques de l'acide azobarbiturique de formule (IIa)

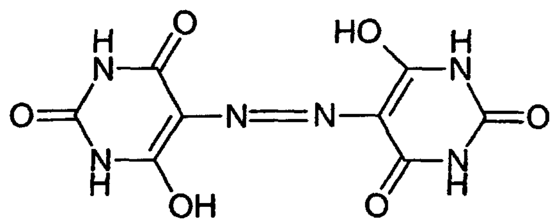

(IIa)

et leurs hydrates.

**2.** Sel monopotassique de l'acide azobarbiturique selon la revendication 1 et ses hydrates.

**3.** Sel monopotassique de l'acide azobarbiturique selon la revendication 2 à l'état de monohydrate.

**4.** Sel monopotassique selon la revendication 2, **caractérisé en ce que** le monohydrate, dans les conditions de la calorimétrie différentielle et à une vitesse de chauffage de 10 K/min, libère un équivalent molaire d'eau de cristallisation à une température de 135 ± 10°C.

**5.** Sel monopotassique selon la revendication 2, **caractérisé en ce que** le monohydrate, dans les conditions de la calorimétrie différentielle, à une vitesse de chauffage de 10 K/min, libère un équivalent molaire d'eau de cristallisation à une température de 230 ± 10°C.

**6.** Sel monopotassique selon la revendication 2, **caractérisé en ce que** l'hydrate, dans les conditions de la calorimétrie différentielle a une vitesse de chauffage de 10 K/min libère 0,4 à 0,6, de préférence 0,5 équivalent molaire d'eau de cristallisation à une température de 195 ± 10°C.

**7.** Procédé pour la préparation des sels mono- ou di-potassiques selon la revendication 1, **caractérisé en ce que** l'on copule le diazo de formule (Va) ou (Vb),

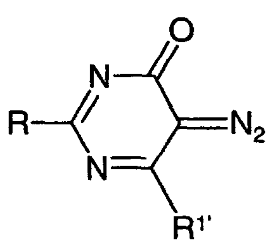

(Va),

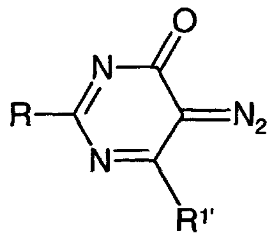

(Vb),

dans laquelle R, $R^1$, R' et $R^{1'}$ représentent chacun un groupe OH, à l'état d'acide libre, en présence de sels de potassium à réaction basique, avec l'acide barbiturique ou ses dérivés de formule (IVa) et/ou (IVb)

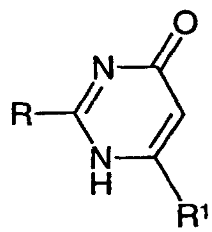

(IVa),

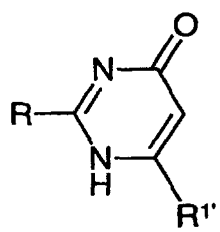

(IVb)

**8.** Procédé selon la revendication 7, **caractérisé en ce que** l'on obtient le diazo d'acide barbiturique en convertissant son sel de sodium en l'acide à un pH inférieur à 1,5 et en isolant cet acide.

**9.** Procédé pour la préparation des dérivés mono- ou di-potassiques selon la revendication 1, **caractérisé en ce que** l'on fait réagir des dérivés de l'acide barbiturique de formules (IVa) et/ou (IVb)

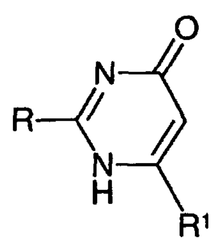

(IVa),

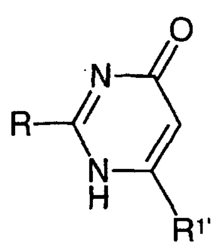

(IVb),

dans lesquelles R, R', $R^1$ et $R^{1'}$ représentent chacun un groupe OH, avec des réactifs apportant un groupe azo, ce qui donne un dérivé d'acide diazobarbiturique qu'on copule en présence de composés du potassium à réaction alcaline sur des dérivés de l'acide barbiturique de formule (IVa) ou (IVb).

**10.** Procédé selon la revendication 9, **caractérisé en ce que** le réactif apportant un groupe azo a été préparé en présence de composés du potassium, plus spécialement du nitrite de potassium, en tant que sources uniques d'alcali.

**11.** Complexes métalliques potassiques d'un dérivé azoïque selon la revendication 1, contenant au moins un composé en inclusion.

**12.** Procédé pour la préparation des complexes métalliques potassiques selon la revendication 11, **caractérisé en ce que** l'on fait réagir le sel mono- ou di-potassique selon la revendication 1 avec un composé à inclure, plus spécialement un composé organique, et en particulier à un pH de 1 à 7.

**13.** Procédé pour la préparation de complexes métalliques dont le métal est choisi dans le groupe consistant en Ca, Zn, Cu, Fe, Cd, Sr, Ba, Cr, Sn, Al, Mg, Pd, La et Ni, **caractérisé en ce que** l'on fait réagir les sels de potassium selon la revendication 1, de préférence à un pH inférieur à 7, et le cas échéant, le complexe métallique ainsi obtenu avec un composé à inclure, de préférence à un pH de 1 à 7.

**14.** Procédé pour la préparation du monohydrate selon la revendication 4, **caractérisé en ce que** l'on copule l'acide diazobarbiturique à l'état d'acide libre et en présence de composés du potassium à réaction basique avec l'acide barbiturique et le cas échéant on isole le monohydrate obtenu.

**15.** Procédé pour la préparation du monohydrate selon la revendication 5, **caractérisé en ce que** l'on chauffe le monohydrate selon la revendication 4 à une température de 140 à 160°C à l'autoclave à un pH de 2 à 6.